**Europäisches Patentamt**

**European Patent Office**

**Office européen des brevets**

(19)

(11) Veröffentlichungsnummer: **0 099 018**
**B1**

(12)

# EUROPÄISCHE PATENTSCHRIFT

(45) Veröffentlichungstag der Patentschrift:
07.01.87

(51) Int. Cl.⁴: **A 61 B 10/00**

(21) Anmeldenummer: **83106247.6**

(22) Anmeldetag: **27.06.83**

(54) **Ultraschall-Tomographiegerät.**

(30) Priorität: **30.06.82 DE 3224453**

(43) Veröffentlichungstag der Anmeldung:
**25.01.84 Patentblatt 84/4**

(45) Bekanntmachung des Hinweises auf die Patenterteilung:
**07.01.87 Patentblatt 87/2**

(84) Benannte Vertragsstaaten:
**DE FR GB NL**

(56) Entgegenhaltungen:
**EP - A - 0 032 751**
**WO - A - 80/00193**
**FR - A - 2 332 531**
**US - A - 4 105 018**
**US - A - 4 130 112**
**US - A - 4 137 777**
**US - A - 4 317 369**

(73) Patentinhaber: **Siemens Aktiengesellschaft Berlin und München, Wittelsbacherplatz 2, D-8000 München 2 (DE)**

(72) Erfinder: **Hassler, Dieter, Flurweg 3, D-8521 Uttenreuth (DE)**
Erfinder: **Trautenberg, Elmar, Dr., Steinacher Strasse 32a, D-8510 Fürth-Stadeln (DE)**

## Beschreibung

Die Erfindung bezieht sich auf ein Ultraschall-Tomographiegerät gemäss den Merkmalen des Oberbegriffs des Patentanspruchs 1.

Ultraschall-Tomographiegeräte lassen sich entweder nach der Transmissions- oder nach der Reflexionsmethode betreiben. Ein Ultraschall-Tomographiegerät für Transmissions-Tomographie (UCTT) ist z.B. aus der US-PS 4 105 018 vorbekannt. Ultraschall-Tomographiegeräte für Reflexions-Tomographie (UCTR) sind beispielsweise bekannt durch den Aufsatz «Resolution and Image Quality by Ultrasonic Echo Tomography: Experimental Approach» von E. Hundt, G. Maderlechner, E. Kronmüller und E. Trautenberg aus «Fifth International Symposium on Ultrasonic Imaging and Tissue Characterization and Second International Symposium on Ultrasonic Materials Characterization», June 1-6, 1980, Seite 7, oder auch durch den Aufsatz «Ultrasonic Reflectivity Tomography: Reconstruction with Circular Transducer Arrays» von Stephen J. Norton und Melvin Linzer aus «Ultrasonic Imaging 1», 1979, Seiten 154–184.

Diese bekannten Ultraschall-Tomographiegeräte erlauben aber bei der Untersuchung z.B. der weiblichen Brust keine brustwandnahe Abtastung im Sinne der Erzeugung eines brustwandnahen Koronarschnittbildes, da wegen der räumlichen Abmessungen das Ultraschall-Sende-/Empfangssystem nicht nahe genug an die Brustwand herangeführt werden kann.

Aufgabe der vorliegenden Erfindung ist es, ein Ultraschall-Tomographiegerät anzugeben, das ohne allzu grossen technischen Aufwand brustwandnahe Koronar-Schnittbilder liefert.

Die Aufgabe wird erfindungsgemäss durch die kennzeichnenden Merkmale des Patentanspruches 1 gelöst.

Die Erfindung ermöglicht den abschnittsweisen Aufbau eines brustwandnahen Koronar-Schnittbildes aus mehreren, leicht gegeneinander geneigten koronaren Einzelschnitten lediglich aufgrund schrittweisen Verstellens des Neigungswinkels der jeweiligen Sende-/Empfangskeule und abschnittsweisen Erfassens von Signaldaten, die sich aufgrund des schrittweisen Verstellens aus den unterschiedlichen Blickrichtungen in den unterschiedlichen Objekttiefen ergeben.

Die Gruppen von Ultraschallköpfen können unterschiedlich ausgebildet sein. So können die Ultraschallköpfe Wandlerelemente umfassen, z.B. nach Art eines Linear-Arrays, Arrays für elektronisch gesteuerten Sektor-Scan, Ring-Arrays od. dgl.. Der Ultraschallkopf kann aber auch einen einzelnen Ultraschallschwinger umfassen, der Bestandteil z.B. eines langsam rotierenden Sektorabtasters ist. Bevorzugte Ausgestaltungen der Erfindung in dieser Art ergeben sich z.B. aus den Unteransprüchen 2 bis 7. In weiterer bevorzugter Ausgestaltung der Erfindung sollten die einzelnen Ultraschallköpfe oder Gruppen von Ultraschallköpfen zur Fokussierung auf unterschiedliche Objekttiefen unterschiedliche Apertur haben.

Weitere Vorteile und Einzelheiten der Erfindung ergeben sich aus der nachfolgenden Beschreibung mehrerer Ausführungsbeispiele anhand der Zeichnung und in Verbindung mit den Unteransprüchen. Es zeigen:

Fig. 1 eine schematische Skizze zur Verdeutlichung der Wirkungsweise der Erfindung;

Fig. 2 ein Prinzipschaltbild zur Anwendung bei der Erfindung;

Fig. 3 ein Ausführungsbeispiel mit mechanischen Sektor-Scannern;

Fig. 4 und 5 ein Ausführungsbeispiel für ein Linear-Array oder Array mit elektronisch erzeugtem Sektor-Scan und

Fig. 6 ein Ausführungsbeispiel mit Ring-Array.

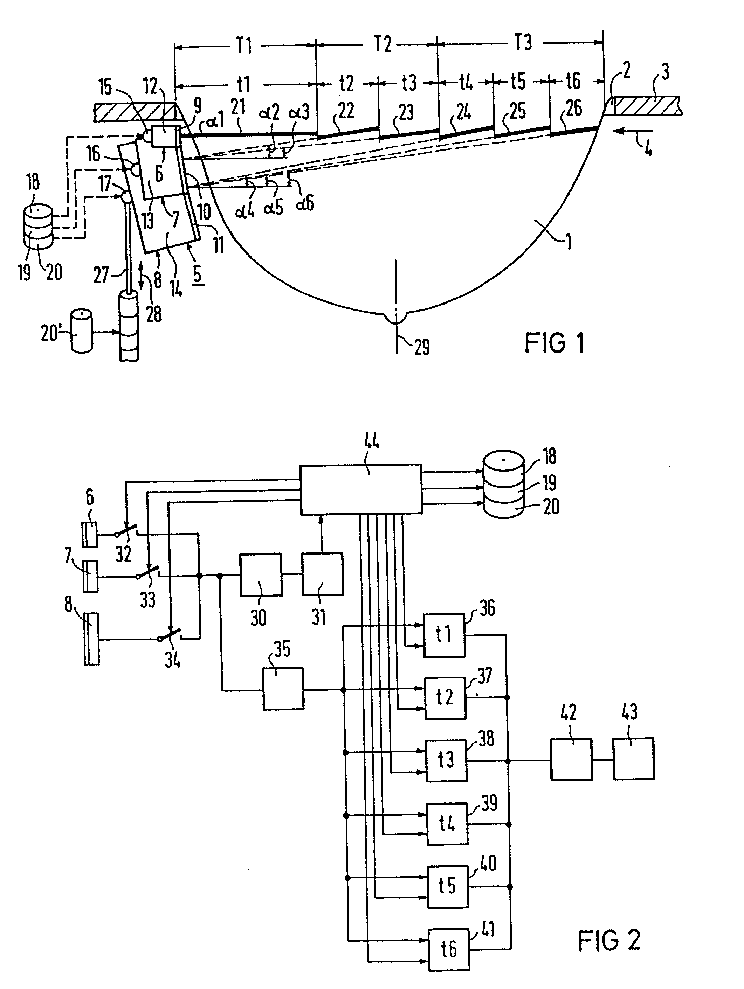

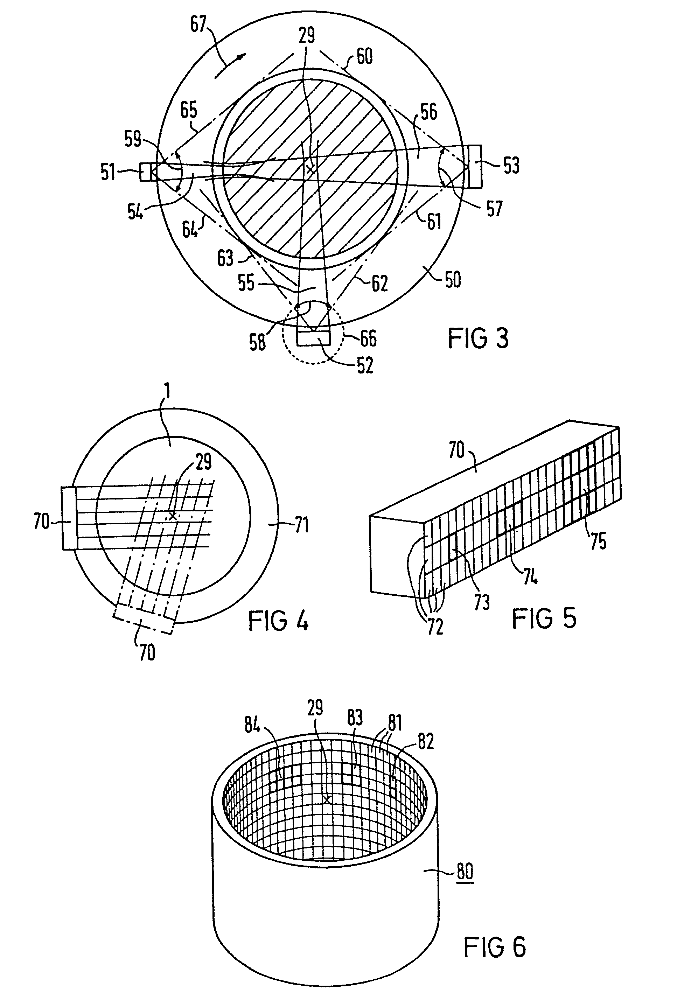

In der Figur 1 ragt eine weibliche Brust 1 als Untersuchungsobjekt durch eine Applikationsöffnung 2 in einer Platte 3, die z.B. Bestandteil eines Patiententisches ist, auf dem die Patientin liegt. Ein Pfeil 4 deutet eine brustwandnahe Schnittebene an, aus der ein Ultraschall-Sende-Empfangssystem 5 ein brustwandnahes Koronar-Schnittbild liefern soll. Das Ultraschall-Sende-/Empfangssystem 5 besteht im vorliegenden Fall z.B. aus drei Ultraschall-Wandlerköpfen oder Schallköpfen 6, 7 und 8, die alle in einem gemeinsamen Gehäuse untergebracht sein können. Genausogut können die Schallköpfe 6, 7, 8 aber auch am Umfang eines Kreises versetzt angeordnet sein, wie dies z.B. in der Figur 3 angedeutet ist. Bei den Ultraschallköpfen 6, 7 und 8 handelt es sich im vorliegenden Fall beispielsweise um mechanische Sektor-Scanner. Sie umfassen Ultraschall-Sende-/Empfangskristalle 9, 10 bzw. 11, die auf Trägerteilen 12, 13, 14 (alles in schematischer Darstellung gezeigt) angeordnet sind.

Erstes wesentliches Merkmal dieser Schallkopf-Formation ist, dass die einzelnen Ultraschallköpfe 6, 7 und 8 an Horizontaldrehgelenken 15, 16, 17 schwenkbar gehaltert sind. Mittels eines motorischen Antriebssystems 18, 19 und 20 lassen sich die einzelnen Schallköpfe 6, 7, 8 relativ zueinander so verstellen, dass sich unterschiedliche Neigungswinkel $\alpha$ bezüglich der angedeuteten brustwandnahen koronaren Abtastebene des zu untersuchenden Objektes, d.h. der weiblichen Brust 1, ergeben. In der Figur 1 beträgt für den ersten Schallkopf 6 der Neigungswinkel beispielsweise $\alpha 1 = 0$. Dazu unterschiedliche Neigungswinkel $\alpha 2$ und $\alpha 3$ bzw. $\alpha 4$ bis $\alpha 6$ sind dem zweiten Schallkopf 7 bzw. dem dritten Schallkopf 8 zugeordnet.

Das ganze System 5 lässt sich mittels eines Rotationsantriebs 20' langsam um eine Drehachse 29, die mit der Symmetrieachse der Brust 1 zusammenfällt, drehen.

Zweites wesentliches Merkmal der Schallkopf-Anordnung ist, dass alle drei Schallköpfe 6, 7, 8 unterschiedliche Apertur haben. Die Apertur des kleinsten Ultraschallkopfes 6 ist so, dass dessen Sende-/Empfangskeule auf den hautnahen Tiefenbereich T1 der weiblichen Brust 1 fokussiert ist. Beim nächstgrösseren Ultraschallkopf 7 ist die Apertur so, dass die Sende-/Empfangskeule auf einen mittleren Tiefenbereich T2 der weiblichen Brust 1 fokussiert ist. Beim Ultraschallkopf 8 mit der grössten Apertur ist die Sende-/Empfangskeule auf den grössten Tiefenbereich T3 der weiblichen Brust 1 fokussiert.

Bei schrittweisem Verstellen der Neigungswinkel α der Wandlerköpfe und gleichzeitiger Rotation des Systems 5 um die Brust 1 sowie gleichzeitiger Betätigung einer Zeittorschaltung (gemäss Figur 2) lassen sich in aufeinanderfolgenden Zeitabschnitten t1, t2, t3, t4, t5 und t6 abschnittsweise Signaldaten aus Bereichsabschnitten 21, 22, 23, 24, 25 und 26 gemäss der Darstellung in Figur 1 erfassen. Die Abschnitte 21 bis 26 nähern sich dabei sehr gut der mit dem Pfeil 4 angedeuteten brustwandnahen Koronar-Schnittebene an. Bei Umrechnung der Daten in einem Rechner ergibt sich das Tomographiebild der brustwandnahen Koronarebene.

Zur Brustwandebene parallele Schnittbildebenen erhält man durch Höhenverschiebung des gesamten Systems 5 über ein Motorantriebssystem 27 in Richtung des Pfeiles 28.

Die Figur 2 zeigt ein Prinzipschaltbild zur Steuerung des Ultraschall-Sende-/Empfangssystems 5 der Figur 1. In diesem Prinzipschaltbild sind die einzelnen Schallköpfe unterschiedlicher Apertur wieder mit 6, 7 und 8 bezeichnet. Die Schallköpfe 6, 7, 8 werden wie üblich von einem Hochfrequenzsender 30 mit vorgeschaltetem Taktgenerator 31 betrieben. Die zeit- und positionsgerechte Ansteuerung der Schallköpfe 6, 7, 8 erfolgt über Ansteuerschalter 32, 33 und 34. Die während der Rotation des Systems 5 aus der Brust 1 empfangenen Ultraschall-Echosignale werden einem Empfangsverstärker 35 und von diesem einer Zeittorschaltung mit Zeittoren 36 bis 41 zugeleitet. Die Zeittore 36 bis 41 lassen nur während den in Fig. 1 dargestellten Zeiten t1 bis t6 abschnittsweise Echosignale zum nachfolgenden Computer 42 durch. Aus den so selektierten Daten errechnet der Computer 42 das angenäherte koronare brustwandnahe Schnittbild, das auf einer Anzeigevorrichtung 43 angezeigt wird. Mit 44 ist eine zentrale Systemsteuereinrichtung bezeichnet.

Wie schon zuvor erwähnt, können die drei Schallköpfe 6, 7 und 8 der Figur 1, die sich z.B. in einer einzigen Dose befinden, auch am Umfang eines Kreises verteilt angeordnet sein, z.B. so, wie es in der Figur 3 dargestellt ist.

In der Fig. 3 sind am Rand einer Ringscheibe 50 als Schallköpfe drei mechanische Sektorabtaster 51, 52 und 53 angeordnet, die zur Neigungsverstellung der Sende-/Empfangskeulen 54, 55 und 56 entsprechend Figur 1 im Neigungswinkel verstellbar sind und die zur Fokussierung auf unterschiedliche Objekttiefen T1, T2 und T3 wieder unterschiedlich grosse Apertur haben. Die Ringscheibe 50 rotiert zusammen mit den mechanischen Sektorabtastern 51, 52 und 53 wieder um die durch die Brust 1 gehende Drehachse 29. Zur Erzeugung der Sektorabtastfelder können die Sektorabtaster 51, 52, 53 eine reine Schwenkbewegung ausführen, wie dies z.B in der Figur 3 durch Schwenkpfeile 57, 58, 59 sowie entsprechend zugehörige (strichpunktiert dargestellte) Begrenzungslinien 60 bis 65 dargestellt ist. Die einzelnen Sektorabtaster 51, 52, 53 können selbstverständlich auch rundum rotierende Abtaster sein, wie dies z.B. in der Figur 3 für den Sektorabtaster 52 durch die punktierte Linie 66 angedeutet ist. Die Rotationsfrequenz der Ringscheibe 50 ist in beiden Fällen sehr viel niedriger als die Frequenz, mit der die Sektorabtaster 51, 52, 53 geschwenkt oder gedreht werden. So beträgt z.B. die Drehgeschwindigkeit der Ringscheibe 50 zusammen mit den Sektorabtastern 0,1 Hz. Die Schwenk- oder Drehfrequenz der Sektorabtaster 51, 52, 53 liegt hingegen bei 3 bis 4 Hz. Die Rotationsbewegung der Ringscheibe 50 ist durch einen Rotationspfeil 67 angedeutet.

Die Figur 4 zeigt ein weiteres Ausführungsbeispiel für gemäss der Erfindung ausgebildetes Ultraschall-Sende-/Empfangssystem. Im Falle der Fig. 4 ist der Ultraschallkopf ein Ultraschall-Array 70 (oder es sind auch mehrere dieser Arrays vorhanden), z.B. ein Linear-Array oder Array mit elektronisch gesteuertem Strahlschwenk für Sektorabtastung, das wieder entlang einem Kreisbogen 71 langsam um die durch die Brust 1 gehende Achse 29 gedreht wird. Eine zweite Drehstellung des Ultraschall-Arrays 70 ist in der Fig. 4 strichpunktiert angedeutet. Das Array 70 ist vorzugsweise ein Mehrzeilen-Array, d.h. ein solches mit matrixartiger Anordnung der einzelnen Wandlerelemente. Die Fig. 5 zeigt beispielsweise ein solches Array 70 mit drei Zeilen von übereinanderliegenden Wandlerelementen 72. Unterschiedliche Aperturen beim Senden und/oder Empfangen erhält man durch übliche elektronische Zu- oder Wegschaltung von Einzelelementen 72 zu unterschiedlich großen Strahlergruppen. In der Fig. 5 ist diese Art von Aperturbildung durch die drei flächenmäßig unterschiedlichen Aperturen 73, 74 und 75 angedeutet.

Die Figur 6 zeigt schliesslich ein als Ring-Array ausgebildetes Ultraschall-Sende-/Empfangssystem mit flächenhafter Anordnung der Wandlerelemente 81. Die Strahlschwenkung und Strahlfortschaltung entlang dem Ring-Array erfolgt rein elektronisch (z.B. entsprechend Figur 5 der US-PS 4 105 018). Die Einstellung unterschiedlicher Aperturen 82, 83 und 84 erfolgt wie üblich wieder durch Zu- oder Wegschaltung von mehreren Wandlerelementen innerhalb einer Gruppe.

Die Ultraschallköpfe der Figuren 1 bis 6 strahlen direkt in das Untersuchungsobjekt, d.h. die Brust 1. Selbstverständlich lassen sich auch Ausführungsformen angeben, bei denen der Ultraschallkopf indirekt über Spiegel od. dgl. in das Untersuchungsobjekt einstrahlt.

Zur Funktion der in den Figuren 1 und 2 gezeigten Anordnungen ist folgendes zu sagen: Die zentrale Systemsteuereinrichtung 44 kann vorzugsweise ein programmierbarer Rechner sein, der mit einer Anzahl von Ausgangs- oder Steueranschlüssen versehen ist. Das Rechnerprogramm kann dabei in der folgenden Art konzipiert sein:

Für das erste wird davon ausgegangen, dass sich alle Ultraschallköpfe 6, 7, 8 in derselben Winkelposition bezüglich der durch die Mamma 1 verlaufenden Achse 29 befinden.

Bei Start des Betriebes wird zunächst die Höhe des Ultraschallkopfes 6 vorgegeben und diese mit Hilfe des Motors 27 eingestellt. Der Ultraschallkopf 6 wird nun so eingestellt, dass er den Sende- und Empfangswinkel α 1 einnimmt. Gleichzeitig wird die Strahlrichtung des zweiten Ultraschallkopfes 7 so eingestellt, dass er den Strahlwinkel α 2 einnimmt, und die Strahlrichtung des dritten Ultraschallkopfes 8 wird so eingerichtet, dass dieser den Winkel α 4 einnimmt. Alle drei Ultraschallköpfe 6, 7 und 8 haben dabei dieselbe Position bezüglich einer zugeordneten Achse, die jeweils durch die Ultraschallköpfe 15, 16 bzw. 17 verläuft, wobei diese drei Achsen jeweils parallel zu dem in Fig. 1 gezeigten Doppelpfeil 28 sind. Diese Position soll als «erste Abtastposition» bezeichnet werden.

In einem ersten Verfahrensschritt wird sodann die zentrale Systemsteuereinrichtung 44 den Schalter 32 schliessen. In einem nachfolgenden Schritt wird die zentrale Systemsteuereinrichtung 44 den Taktimpulsgenerator 31 anstossen. Dieser wiederum sorgt über den Hochfrequenzübertrager 30 dafür, dass der Ultraschallkopf 6 einen Ultraschallimpuls abstrahlt. In einem nächsten Verfahrensschritt wird die zentrale Systemsteuereinrichtung 44 nun die Torschaltung 36 aktivieren. Daraufhin wird das Echosignal aus der Tiefenregion 21, welche dem Zeitabschnitt $t_1$ entspricht, vom ersten Ultraschallkopf 6 und vom Eingangsverstärker 35 empfangen. Das Echosignal wird an das Zeittor 36 weitergeleitet und anschliessend im Rechner 42 gespeichert, und zwar entsprechend den zugehörigen Koordinaten innerhalb der Abtastebene. In einem nächsten Verfahrensschritt wird dann die zentrale Systemsteuereinrichtung 44 den Schalter 32 öffnen und den Schalter 33 schliessen. Daraufhin wird die zentrale Systemsteuereinrichtung 44 wiederum den Taktimpulsgenerator 31 anstossen. Das Ergebnis hiervon ist, dass der Ultraschallkopf 7 nunmehr einen Ultraschallimpuls in der Winkelrichtung α 2 abgibt. Als nächstes wird die zentrale Systemsteuereinrichtung 44 das Zeittor 37 öffnen. Dadurch wird das zugeordnete Echosignal dieses Zeittor 37 passieren und zum Rechner 42 gelangen. Mit anderen Worten, das Echosignal aus der Tiefenregion 22, welches dem Zeitabschnitt $t_2$ entspricht, wird nunmehr im Rechner 42 gespeichert, und zwar ebenfalls entsprechend den zugehörigen Koordinaten innerhalb der Abtastebene.

In einem weiteren Verfahrensschritt wird die zentrale Systemsteuereinrichtung 44 sodann den Schalter 33 öffnen und dafür den Schalter 34 schliessen. Als Ergebnis hiervon gibt nun der Ultraschallkopf 8 einen Ultraschallimpuls ab, sobald der Taktimpulsgenerator 31 wieder getriggert wird. Anschliessend wird die Systemsteuereinrichtung 44 das Zeittor 39 öffnen. Diesem Zeittor 39 ist der Zeitabschnitt $t_4$ zugeordnet. Das von dem Ultraschallimpuls herrührende Echosignal wird während dieses Zeitabschnitts $t_4$ vom Ultraschallkopf 8 empfangen und anschliessend durch eben dieses Zeittor 39 an den Rechner 42 weitergeleitet. Dieses Echosignal stammt aus einer Tiefenregion, welche dem Zeitabschnitt $t_4$ entspricht. Auch dieses Echosignal wird im Speicher des Rechners 42 gespeichert, und zwar nach Massgabe der Abtastkoordinaten.

Als nächstes sorgt die zentrale Systemsteuereinrichtung 44 dafür, dass der Ultraschallkopf 7 den Abstrahl- und Empfangswinkel α 3 einnimmt. Sie sorgt auch dafür, dass der Ultraschallkopf 8 in der Weise eingestellt wird, dass dieser Ultraschallkopf 8 Ultraschallwellen in und aus einer Winkelrichtung α 5 aussenden bzw. empfangen kann. Nun wird der Schalter 33 geschlossen, und die zentrale Systemsteuereinrichtung 44 sorgt dafür, dass jetzt der Ultraschallkopf 7 einen Ultraschallimpuls abstrahlt. Das resultierende Echosignal wird während des Zeitabschnitts $t_3$ über das Zeittor 38 empfangen, welches zuvor natürlich aktiviert wurde. Dieses Echosignal, das sich nunmehr auf die Tiefenregion 23 bezieht, wird an den Rechner 42 weitergeleitet und ebenfalls darin gespeichert.

Während des nächsten Daten-Akquisitions-Zyklus ist der Schalter 33 geöffnet und der Schalter 34 geschlossen. Ein Ultraschallimpuls wird durch den Ultraschallkopf 8 in Winkelrichtung α 5 abgestrahlt, und das sich ergebende Echosignal wird während der Zeitperiode $t_5$ über das Zeittor 40 empfangen. Auch dieses Echosignal wird im Rechner 42 koordinatengerecht gespeichert.

Im folgenden Zyklus nimmt der Ultraschallkopf 8 die Winkelposition α 6 ein. Der Pulsgenerator 31 wird wiederum getriggert, woraufhin der Ultraschallkopf 8 einen Ultraschallimpuls in das zu untersuchende Objekt 1 abstrahlt. Die zentrale Systemsteuereinrichtung 44 öffnet nun das Zeittor 41, so dass ein Echosignal aus der Tiefenregion 26, welches dem Zeitintervall $t_6$ entspricht, passieren kann. Dieses Echosignal aus der Tiefenregion 26 wird vom Rechner 42 empfangen und an einer geeigneten Stelle in dessen Speicher eingespeichert.

Es ist somit ersichtlich, dass im Verlauf dieses Verfahrens eine ganze Anzahl von Daten im Rechner 42 gespeichert werden. In einem nächsten grundsätzlichen Verfahrensschritt werden dann alle drei Ultraschallköpfe 6, 7, 8 einen Winkelschritt weitergedreht, und zwar um die zuvor erwähnten Achsen, die jeweils parallel zum Doppelpfeil 28 liegen. Jede dieser drei Achsen steht dabei senkrecht auf der zugehörigen Abtastebene. Auf diese Weise nehmen die drei Ultraschallköpfe 6, 7 und 8 eine «zweite Abtastposition» ein. In dieser «zweiten Abtastposition» wird dann die gesamte Prozedur, wie sie oben beschrieben ist, Schritt für Schritt wiederholt. Die während dieser einzelnen Verfahrensschritte erhaltenen Daten werden ebenfalls im Rechner 42 gespeichert.

Es soll hier angemerkt werden, dass in einem typischen Anwendungsfall zwischen 50 und 200 verschiedene «Abtastpositionen» eingenommen werden können. Mit anderen Worten, es kann zwischen 50 und 200 verschiedene «Abtastpositionen» geben, die um vorgegebene Winkelschritte in bezug auf die Achsen, die parallel zum Doppelpfeil 28 sind, variieren.

Nachdem nun in all diesen 50 bis 200 Abtastpositionen die entsprechenden Daten aufgenommen worden sind, werden die Ultraschallköpfe 6, 7 und 8 einschliesslich der Abtasteinrichtungen 15, 16, 17, 27 um einen kleinen Winkelschritt um die Achse 29 gedreht, und dann wird die gesamte Abtastprozedur nach dem oben diskutierten Schema wiederholt.

Insgesamt können pro Koronarschritt 20 bis 200 kleine Winkelschritte bezüglich der Achse 29 eingenommen werden, so dass das zu untersuchende Objekt 1 aus allen Richtungen untersucht wird. In jeder dieser Winkelpositionen werden die Abtastprozeduren wie zuvor beschrieben durchgeführt. Die auf diese Weise aufgenommenen Daten werden ebenfalls im Rechner 42 gespeichert.

Schliesslich bestimmt der Rechner 42 ein Reflexions-CT-Bild von all diesen Daten in an sich bekannter Weise, und dieses CT-Bild wird sodann auf dem Bildschirm des Darstellungsgerätes 43 dargestellt.

Abweichend von dem voranstehend beschriebenen Verfahren kann auch folgendermassen vorgegangen werden, wodurch sich eine Zeiteinsparung bei der mechanischen Einstellung der Ultraschallköpfe 6, 7 und 8 ergibt:

1. Zunächst wird für die Ultraschallköpfe 6, 7, 8 ein bestimmter Winkel $\alpha 1$, $\alpha 2$ bzw. $\alpha 4$ fest vorgegeben. Für diese festen Winkel $\alpha 1$, $\alpha 2$ und $\alpha 4$ wird die gesamte Bildinformation entsprechend den Tiefenbereichen 21, 22 und 24 gewonnen und abgespeichert. Unter gesamter Bildinformation ist dabei diejenige Information zu verstehen, die beim Abtasten zunächst um die Achse 27 und danach um die Achse 29 gewonnen wird. Das Abtasten um die Achse 29 kann dabei – wie im voranstehenden Verfahren auch – winkelschrittweise oder kontinuierlich erfolgen.

2. Danach wird für den Ultraschallkopf 7 statt des Winkels $\alpha 2$ der neue Winkel $\alpha 3$ vorgegeben, und für den Ultraschallkopf 8 wird statt des Winkels $\alpha 4$ der neue Winkel $\alpha 5$ eingestellt. Mit dieser Einstellung wird nun die gesamte Bildinformation aus den Tiefenbereichen 23 und 25 gewonnen und abgespeichert, und zwar in der voranstehend geschilderten Weise. Auf die Abtastung des Tiefenbereichs 26 kann in diesem Fall verzichtet werden, weil die Information für diesen Tiefenbereich 26 bei Umdrehung von 360° um die Achse 29 mit Hilfe des Ultraschallwandlers 6 ohnehin anfällt.

**Patentansprüche**

1. Ultraschall-Tomographiegerät mit einem Ultraschall-Sende-/Empfangssystem (5), das einzelne Ultraschallköpfe (6, 7, 8; 51, 52, 53) oder Gruppen von Ultraschallköpfen (70; 80) aufweist, das Ultraschallpulse aussendet, das zur Abtastung eines Untersuchungsobjektes (1) aus unterschiedlichen Winkelrichtungen in einer koronaren Abtastebene (4) ausgebildet ist und das ein Koronarschnittbild der Abtastebene (4) liefert, dadurch gekennzeichnet, dass die Ultraschallköpfe (6, 7, 8; 51, 52, 53) oder Gruppen von Ultraschallköpfen (70, 80) so ausgebildet sind, dass sie das Untersuchungsobjekt (1) aus den unterschiedlichen Winkelrichtungen zeilenweise abtasten und in der koronaren oder einer koronarähnlichen Abtastebene (4) auf unterschiedliche Objekttiefen (T1, T2, T3) fokussierbar sind, und dass den Ultraschallköpfen (6, 7, 8; 51, 52, 53) oder Gruppen von Ultraschallköpfen (70, 80) eine Verstelleinrichtung (15 bis 20) zum Verstellen des Neigungswinkels ($\alpha 1$ bis $\alpha 6$) der jeweiligen Sende-/Empfangskeule bezüglich der koronaren Abtastebene (4) sowie eine Zeittorschaltung (36 bis 41) zum abschnittweisen Erfassen (21 bis 26) von Signalen aus den unterschiedlichen Objekttiefen (T1, T2, T3) zugeordnet sind, so dass eine Abtastzeile in der Abtastebene (4) aus Bereichsabschnitten (21 bis 26) zusammensetzbar ist, die in der Abtastebene (4) liegen oder hierzu leicht geneigt sind.

2. Ultraschall-Tomographiegerät nach Anspruch 1 dadurch gekennzeichnet, dass eine vorgegebene Zahl der Schallköpfe, z.B. auch nur ein einzelner Schallkopf (70), die mit einer Mehrzal von gruppenweise ansteuerbaren Wandlerelementen (72) bestückt ist, im Sinne der zeilenweisen Abtastung des Untersuchungsobjektes (1) aus unterschiedlichen Winkelrichtungen ausgebildet ist.

3. Ultraschall-Tomographiegerät nach Anspruch 2, dadurch gekennzeichnet, dass der Schallkopf (70) ein Linear-Array ist, das das Untersuchungsobjekt (1) im Linear-Scan abtastet und das sich zusätzlich um das Untersuchungsobjekt (1) dreht.

4. Ultraschall-Tomographiegerät nach Anspruch 2, dadurch gekennzeichnet, dass der Schallkopf (70) ein Array für elektronisch gesteuerten Sektor-Scan ist, das sich um das Untersuchungsobjekt (1) dreht.

5. Ultraschall-Tomographiegerät nach Anspruch 2, dadurch gekennzeichnet, dass der Schallkopf (80) ein Ring-Array ist, das im Sinne der Erzeugung eine Sektor-Scans und im Sinne der Fortschaltung des Sektor-Scans entlang dem Ring elektronisch steuerbar ist.

6. Ultraschall-Tomographiegerät nach Anspruch 1, dadurch gekennzeichnet, dass ein einzelner oder eine Gruppe mechanischer Sektor-Abtaster (51 bis 53), der (die) nur mit einem einzelnen oder mit einer Mehrzahl von gruppenweise ansteuerbaren Wandlerelementen bestückt ist (sind), im Sinne der zeilenweise Abtastung des Untersuchungsobjektes (1) aus unterschiedlichen Winkelbereichen ausgebildet ist (sind).

7. Ultraschall-Tomographiegerät nach Anspruch 6, dadurch gekennzeichnet, dass zur zeilenweisen Abtastung aus unterschiedlichen Winkelrichtungen dem Sektorschwenk (57, 58, 59) eine Rotation des Sektorabtasters (51 bis 53) um eine Drehachse (29) um das Untersuchungsobjekt (1) überlagert ist.

8. Ultraschall-Tomographiegerät nach einem der Ansprüche 1 bis 7, dadurch gekennzeichnet, dass die einzelnen oder Gruppen von Ultraschallköpfen (6, 7, 8; 51, 52, 53; 70; 80) zur Fokussierung auf unterschiedliche Objekttiefen (T1, T2, T3) unterschiedliche Apertur haben.

9. Ultraschall-Tomographiegerät nach Anspruch 8, dadurch gekennzeichnet, dass zur Bildung der unterschiedlichen Aperturen entweder die Schallköpfe (6, 7, 8; 51, 52, 53) selbst unterschiedliche Sende-/Empfangsflächen haben (Figuren 1 und 3), oder dass bei Schallköpfen (70; 80) mit konstanten äusseren Abmessungen und matrixartiger Anordnung der Wandlerelemente (72; 81) (Figuren 4, 5, 6) eine vorgegebene Zahl von Ultraschall-Wandlerelementen (73, 74, 75; 82, 83, 84) zu unterschiedlichen Sende-/Empfangsflächen (73, 74, 75; 82, 83, 84) zusammenschaltbar ist.

10. Ultraschall-Tomographiegerät nach einem der Ansprüche 1 bis 9, dadurch gekennzeichnet, dass die Verstelleinrichtung zum Verstellen des Neigungswinkels ($\alpha$) der Sende-/Empfangskeulen des jeweiligen Ultraschall-Sende-/Empfangssystems (5) mechanischer Art ist, so dass die Ultraschallwandler (6, 7, 8) selbst durch entsprechende mechanische Schrägstellung in ihrem Neigungswinkel ($\alpha$) änderbar sind, oder dass die Verstelleinrichtung elektronischer Art ist, die bei gleichbleibendem Stellungswinkel ($\alpha$) des Ultraschall-Sende-Empfangssystems (5) den Neigungswinkel ($\alpha$) der jeweiligen Sende-/Empfangskeule elektronisch verstellt, oder aber dass die Verstelleinrichtung eine Mischform aus beiden genannten Verstelleinrichtungen ist, die also sowohl mechanisch als auch elektronisch verstellt.

**Claims**

1. Ultrasonic tomography apparatus comprising an ultrasonic transmitting/receiving system (5) which possesses individual ultrasonic heads (6, 7, 8; 51, 52, 53) or groups of ultrasonic heads (70; 80), transmitting ultrasonic pulses in a coronal scanning plane (4) to scan an object under examination (1) from different angle directions and supplies a coronal sectional view of the scanning plane (4), characterised in that the ultrasonic heads (6, 7, 8; 51, 52, 53) or groups of ultrasonic heads (70, 80) are such that they scan the object under examination (1) line by line from the different angular directions and can be focussed to different object depths (T1, T2, T3) in the coronal or a coronal-like scanning plane (4), and that the ultrasonic heads (6, 7, 8; 51, 52, 53) or groups of ultrasonic heads (70, 80) are assigned an adjusting device (15 to 20) for adjusting the angle of tilt ($\alpha 1$ to $\alpha 6$) of the respective transmitting/receiving lobe in respect of the coronal scanning plane (4) and a time gate circuit (36 to 41) for the section-wise detection (21 to 26) of signals from the different object depths (T1, T2, T3), so that a scan line in the scanning plane (4) can be composed of area sections (21 to 26) which are arranged in the scanning plane (4) or are slightly tilted thereto.

2. Ultrasonic tomography apparatus as claimed in Claim 1, characterised in that a predetermined number of ultrasonic heads, e.g. even only one individual ultrasonic head (70) equipped with a plurality of transducer elements (72) controlled in groups, and is designed in accordance with the line-wise scanning of the object under examination (1) from different angular directions.

3. Ultrasonic tomography apparatus as claimed in Claim 2, characterised in that the ultrasonic head (7) is a linear array which scans the object under examination (1) in the linear scan and additionally rotates about the object under examination (1).

4. Ultrasonic tomography apparatus as claimed in Claim 2, characterised in that the ultrasonic head (70) is in array for electronically controlled sector scan which rotates about the object under examination (1).

5. Ultrasonic tomography apparatus as claimed in Claim 2, characterised in that the ultrasonic head (80) is a ring array electronically controllable in accordance with the production of a sector scan and in accordance with the advancing operation of the sector scan along the ring.

6. Ultrasonic tomography apparatus as claimed in Claim 1, characterised in that an individual or a group of mechanical sector scanners (51 to 53), which is/are only equipped with one individual or a plurality of transducer elements controllable in groups, is designed in accordance with the line-wise scanning of the object under examination (1) from different angular directions.

7. Ultrasonic tomography apparatus as claimed in Claim 6 characterised by line-wise scanning from different angle directions, a rotation of the sector scanner (51 to 53) about an axis of rotation (29) around the object under examination (1) being superimposed upon the sector swivel (57, 58, 59).

8. Ultrasonic tomography apparatus as claimed in one of Claims 1 to 7, characterised in that the individual or groups of ultrasonic heads (6, 7, 8; 51, 52, 53; 70, 80) have a different aperture for focussing onto different object depths (T1, T2, T3).

9. Ultrasonic tomography apparatus as claimed in Claim 8, characterised in that in order to form the different apertures either the ultrasonic heads (6, 7, 8; 51, 52, 53) themselves have different transmitting/receiving surfaces (Figures 1 and 3) or that in ultrasonic heads (70; 80) with constant outer dimensions and a matrix-like arrangement of the transducer elements (72; 81) (Figures 4, 5, 6) a predetermined number of ultrasonic transducer elements (73, 74, 75; 82, 83, 84) can be switched togehter to form different transmitting /receiving surfaces (7, 74, 75; 82, 83, 84).

10. Ultrasonic tomography apparatus as claimed in one of Claims 1 to 9, characterised in that the adjusting device for adjusting the angle of tilt ($\alpha$) of the transmitting/receiving system (5) is of a mechanical type, so that the ultrasonic transducers (6, 7, 8) themselves can be changed by corresponding mechanical inclination in their angle of tilt ($\alpha$) or that the adjusting device is of an electronic type which electronically adjusts the angle of tilt ($\alpha$) of the respective transmitting/receiving lobe in the event of a constant setting angle ($\alpha$) of the ultrasonic transmitting/receiving system (5), or that the adjusting device is a combination of said two adjusting devices which adjusts both mechanically and also electronically.

**Revendications**

1. Appareil de tomographie par ultrasons avec un système émission/réception d'ultrasons (5), qui comporte des têtes ultrasonores individuelles (6, 7, 8; 51, 52, 53) ou des groupes de têtes ultrasonores (70; 80), qui émet des impulsions ultrasonores, qui est réalisé pour balayer un objet à examiner (1) à partir de différentes directions angulaires dans un plan de balayage coronaire (4) et qui fournit une image coronaire tomographique du plan de balayage (4), caractérisé par le fait que les têtes ultrasonores (6, 7, 8; 51, 52, 53) ou les groupes de têtes ultrasonores (70, 80) qui sont réalisés de telle façon qu'ils balaient ligne par ligne, à partir des différentes directions angulaires et sont susceptibles d'être focalisés sur des profondeurs différentes (T1, T2, T3) de l'objet, dans le plan coronaire de balayage ou dans un plan de balayage simili-coronaire (4), et qu'aux têtes ultrasonores (6, 7, 8; 51, 52, 53) ou groupes des têtes ultrasonores (70, 80) sont associés un dispositif de déplacement (15 à 20) pour ajuster l'angle d'inclinaison ($\alpha$1 à $\alpha$6) des lobes d'émission/de réception par rapport au plan coronaire de balayage (4), ainsi qu'un circuit logique à commande par horloge (36 à 41) pour saisir section par section (21 à 26) des signaux à partir des différentes profondeurs (T1, T2, T3) de l'objet, en sorte qu'une ligne de balayage est constituée, dans le plan de balayage (4), par des sections de zones (21 à 26) qui se situent dans le plan de balayage (4) et qui sont légèrement inclinées par rapport à ce dernier.

2. Appareil de tomographie par ultrasons selon la revendication 1, caractérisé par le fait qu'un nombre prédéterminé de têtes ultrasonores, par exemple également une seule tête ultrasonore (70), et qui est pourvu de plusieurs transducteurs (72) qui sont susceptibles d'être attaqués par groupes, est réalisé pour le balayage, ligne par ligne, de l'objet à éxaminer (1), suivant différentes directions angulaires.

3. Appareil de tomographie par ultrasons selon la revendication 2, caractérisé par le fait que la tête ultrasonore (70) est un système linéaire qui balaie linéairement l'objet à examiner (1) et qui, de plus, tourne autour de ce dernier.

4. Appareil de tomographie par ultrasons selon la revendication 2, caractérisé par le fait que la tête ultrasonore (70) est un système de balayage par secteurs à commande électronique, qui tourne autour de l'objet à examiner (1).

5. Appareil de tomographie par ultrasons selon la revendication 2, caractérisé par le fait que la tête ultrasonore (80) est un système annulaire qui, pour produire un balayage par secteurs et pour faire avancer pas-à-pas le balayage par secteurs, est susceptible d'être commandé électroniquement le long de l'anneau.

6. Appareil de tomographie par ultrasons selon la revendication 1, caractérisé par le fait qu'un seul dispositif mécanique de balayage par secteur et un groupe de dispositifs mécaniques de balayage par secteurs (51 à 53) qui est, ou qui sont pourvus d'un seul ou de plusieurs transducteurs qui sont susceptibles d'être attaqués par groupes, est ou sont réalisés pour le balayage ligne par ligne de l'objet à examiner (1) suivant différentes plages angulaires.

7. Appareil de tomographie par ultrasons selon la revendication 6, caractérisé par le fait que pour le balayage ligne par ligne suivant des directions angulaires différentes, on superpose au basculement sectoriel (57, 58, 59) une rotation du dispositif de balayage sectoriel (51 à 53) autour d'un axe de rotation (29) autour de l'objet à examiner. (1).

8. Appareil de tomographie par ultrasons selon l'une des revendications 1 à 7, caractérisé par le fait que les différentes têtes ultrasonores ou les groupes de têtes ultrasonores (6, 7, 8; 51, 52, 53; 70; 80) possèdent, pour la focalisation sur des profondeurs différentes (T1, T2, T3) de l'objet, des ouvertures différentes.

9. Appareil de tomographie par ultrasons selon la revendication 8, caractérisé par le fait que pour former les différentes ouvertures, les têtes ultrasonores (6, 7, 8; 51, 52, 53) possèdent elles-mêmes des surfaces d'émission/de réception différentes (figures 1 et 3), ou, dans le cas de têtes ultrasonores (70; 80) à dimensions extérieures constantes et à disposition matricielle des transducteurs (72; 81) (figures 4, 5, 6), un nombre prédéterminé de transducteurs ultrasonores (73, 74, 75; 82, 83, 84) est susceptible d'être assemblé pour former des surfaces d'émission/de réception différentes (73, 74, 75; 82, 83, 84).

10. Appareil de tomographie par ultrasons selon l'une des revendications 1 à 9, caractérisé par le fait que le dispositif de déplacement pour ajuster l'angle d'inclinaison ($\alpha$) des lobes d'émission/de réception du système ultrasonore d'émission/de réception (5), est du type mécanique, en sorte que l'angle d'inclinaison ($\alpha$) des transducteurs ultrasonores (6, 7, 8) est susceptible d'être modifié par un positionnement mécanique oblique, ou que le dispositif de déplacement est du type électronique qui, pour un angle de réglage ($\alpha$) inchangé du système ultrasonore d'émission/de réception (5), déplace électroniquement l'angle d'inclinaison ($\alpha$) du lobe d'émission/de réception concerné, ou que le dispositif de déplacement est un mélange des deux dispositifs de déplacement procédant à un réglage aussi bien mécanique qu'électronique.

FIG 1

FIG 2

FIG 3

FIG 4

FIG 5

FIG 6